(19) Europäisches Patentamt
European Patent Office
Office européen des brevets

(11) Veröffentlichungsnummer: **0 149 832**
**B1**

(12) # EUROPÄISCHE PATENTSCHRIFT

(45) Veröffentlichungstag der Patentschrift: **25.04.90**

(51) Int. Cl.[5]: **C 07 C 333/02**

(21) Anmeldenummer: **84115943.7**

(22) Anmeldetag: **20.12.84**

(54) **Verfahren zur Herstellung von Thiolcarbaminsäureestern.**

(30) Priorität: **29.12.83 HU 450983**
**29.12.83 HU 451083**
**10.12.84 HU 458384**
**10.12.84 HU 458484**

(43) Veröffentlichungstag der Anmeldung:
**31.07.85 Patentblatt 85/31**

(45) Bekanntmachung des Hinweises auf die Patenterteilung:
**25.04.90 Patentblatt 90/17**

(84) Benannte Vertragsstaaten:
**BE CH DE FR GB IT LI NL SE**

(56) Entgegenhaltungen:
**CHEMICAL ABSTRACTS, Band 92, Nr. 21, 26. Mai 1980, Seite 613, Nr. 180759k, Columbus, Ohio, US; B.A. TROFIMOV et al.: "Addition of dithiocarbamate anion to acetylene and phenylacetylene"**

(73) Patentinhaber: **MTA Központi Kémiai Kutato Intézete**
**Pusztaszeri u. 59/67**
**H-1025 Budapest (HU)**

(73) Patentinhaber: **BORSODI VEGYI KOMBINAT**
**Bolyai tér 1**
**H-3702 Kazincbarcika (HU)**

(72) Erfinder: **Besenyei, Gábor, Dipl.-Chem.**
**Bocskay u. 106**
**1153 Budapest (HU)**
Erfinder: **Viski, Péter, Dipl-Chem.**
**Pozsonyi u. 25**
**1137 Budapest (HU)**
Erfinder: **Simándi, László, Dipl.-Chem.**
**Ipoly u. 16**
**1133 Budapest (HU)**
Erfinder: **Nagy, Ferenc, Dipl.-Chem.**
**Mexikói ut 54**
**1145 Budapest (HU)**
Erfinder: **Pászty, György, Dipl.-Ing.**
**Hegyköz u. 6**
**3702 Kazincbarcika (HU)**
Erfinder: **Pázmándi, Gyula, Dipl.-Ing.**
**Bólyai tér 9**
**3702 Kazincbarcika (HU)**

EP 0 149 832 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

(72) Erfinder: Szita, István, Dipl.-Ing.
Középszer u. 88
3529 Miskolc (HU)
Erfinder: Szilágyi, Gyula, Dipl.-Chem.
Benedek u. 15
3534 Miskolc (HU)
Erfinder: Tóth, István
Mártirok u. 26
1025 Budapest (HU)
Erfinder: Szigeti, Ilona, geb. Kiss
Csobánka tér 2
1039 Budapest (HU)

(74) Vertreter: von Füner, Alexander, Dr. et al
Patentanwälte v. Füner, Ebbinghaus, Finck
Mariahilfplatz 2 & 3
D-8000 München 90 (DE)

**Beschreibung**

Die Erfindung betrifft ein neues Verfahren zur Herstellung der bekannten Thiolcarbaminsäureester der allgemeinen Formel (I)

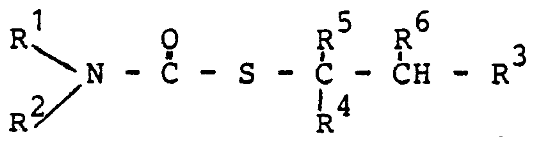
(I)

worin

$R^1$ und $R^2$ unabhängig voneinander für Wasserstoff oder eine gerade oder verzweigte Alkylgruppe mit 1—6 Kohlenstoffatomen oder Alkenylgruppe mit 2—6 Kohlenstoffatomen oder für durch Halogen, Sauerstoff, Schwefel und/oder Stickstoff ein- oder mehrfach substituierte Alkylgruppe mit 1—6 beziehungsweise Alkenylgruppe mit 2—6 Kohlenstoffatomen stehen oder zusammen eine gegebenenfalls substituierte α,ω-Alkylengruppe mit 4—6 Kohlenstoffatomen bilden,

$R^3$ und $R^4$ unabhängig voneinander für Wasserstoff oder eine Alkylgruppe mit 1—4 Kohlenstoffatomen stehen und letztere durch Halogen oder eine Sauerstoff, Schwefel und/oder Stickstoff enthaltende Gruppe substituiert sein kann, und

$R^5$ und $R^6$ beide für Wasserstoff stehen oder gemeinsam eine chemische Bindung bilden.

Abhängend von der Bedeutung von $R^5$ und $R^6$ handelt es sich bei den erfindungsgemäß herstellbaren Estern um Thiolcarbaminsäure-S-alkenylester ($R^5$ und $R^6$ = chemische Bindung) oder um Thiolcarbaminsäure-S-alkylester ($R^5 = R^6 = H$). Erstere werden in der Kunststoffindustrie zur Herstellung polymerer Produkte und in der chemischen Industrie als Intermediäre in der Herstellung von Pflanzenschutzmitteln verwendet, letztere in der Landwirtschaft als Wirkstoffe von Pflanzenschutzmitteln.

Eine verbreitet eingesetzte Methode zur Herstellung von Vinylverbindungen besteht darin, eine beweglichen Wasserstoff enthaltende Verbindung an Acetylen zu addieren. Ober die Anwendungsmöglichkeiten dieser Methode gibt W. Reppe (Ann. *601*, 81—138/1956/) einen Überblick. Der Autor beschreibt die Herstellung zahlreicher Vinylverbindungen, Alkenylester der Thiolcarbaminsäure werden jedoch nicht erwähnt.

Zur Herstellung der S-(1-Alkenyl)-ester von Monothiocarbaminsäuren arbeiteten als Erste C. G. Overberger, H. Ringsdorf und N. Weinshenker eine Methode aus (J. Org. Chem. *27*, 4331—37/1962/). Das Wesen ihres Verfahrens besteht darin, daß aus dem vorher bereiteten Monothiocarbaminsäure-S-(β-chloräthyl)-ester mit Kalium-tert.-butylat Salzsäure abgespalten wird. Obwohl die Umsetzung mit guter Ausbeute ausgeführt werden kann, hat sich doch das Verfahren in der Praxis nicht durchgesetzt, weil die Ausgangsstoffe teuer, korrosiv und giftig sind.

Die Thiolcarbaminsäurealkylester der allgemeinen Formel (I) werden in der Praxis erhalten, indem man das primäre, sekundäre oder tertiäre Ammoniumsalz oder Alkalisalz der entsprechenden Thiolcarbaminsäure mit einem Alkylhalogenid umsetzt (sowjetische Patentschriften Nr. 224 511 und 186 437, DT—PS Nr. 2 513 196 und 2 844 305, japanische Patentschriften 75 76026, 78 25564, 77 78832, 77 746027, US—PS Nr. 3 167 571 und 3 151 119).

Gemäß den DT—PS Nr. 2 212 766 und 2 117 115 und US—PS Nr. 4 066 081 werden Alkylmercaptane mit Phosgen umgesetzt und das erhaltene Alkyl-(chlor-thioformiat) mit primären oder sekundären Aminen zur Reaktion gebracht.

Die Alkylthiolcarbamate können auch durch Umsetzung von Carbamoylchlorid mit Alkylmercaptanen hergestellt werden (US—PS Nr. 2 983 747 und 2 913 327 sowie spanische Patentschrift Nr. 422 149).

Gemäß der DT—PS Nr. 2 703 106 werden Thiolcarbamate durch Einwirkung von Dimethylsulfat und elementarem Jod auf Dithiocarbamate erhalten.

Gemäß der DT—PS Nr. 2 461 876 werden O-Alkylthiolcarbamate mit Dialkylsulfaten erhitzt, wobei sich durch Isomerisation S-Alkylester bilden.

Gemäß US—PS Nr. 4 071 423 werden Thiolcarbamate in außerordentlich bescheidener Ausbeute durch Radikaladdition von Olefinen und den Salzen von Thiolcarbaminsäuren gewonnen.

Beschreibungseinschub auf Seite 3 nach Zeile 21

Alle diese Verfahren erfordern teure Ausgangsstoffe oder sind (Verwendung von Phosgen) eine Belastung für den Gesundheits- und Umweltschutz.

Die Aufgabe der Erfindung bestand nun darin, ein neues, vorteilhaftes Verfahren zur Herstellung der Thiolcarbaminsäure-S-alkyl-beziehungsweise-S-alkenylester der allgemeinen Formel (I) zu finden.

Chem. Abs. 92, 1980, Nr. 180759K betrifft die Herstellung von Dithiocarbamidsäure-S-vinylestern durch die Umsetzung von Natrium- oder Kaliumdithiocarbamat und Acetylen (oder Phenylacetylen) in einem dipolar-aprotischen Medium.

Die Erfindung beruht auf der Erkenntnis, daß die ungesättigten Verbindungen der allgemeinen Formel (I) durch nukleophile Addition von 1-Alkinen, zum Beispiel Acetylen, an Thiolcarbaminsäuresalze hergestellt werden können. Erkannt wurde ferner, daß es nicht unbedingt erforderlich ist, die

Thiolcarbaminsäuresalze vorher herzustellen und zu isolieren, sondern dass sie in situ aus dem entsprechenden Amin, Schwefel und Kohlenmonoxyd beziehungsweise dem entsprechenden Amin und Carbonylsulfid erzeugt und in dieser Form mit dem Alkin umgesetzt werden können.

Die Erfindung beruht ferner auf der Erkenntnis, daß die Thiolcarbaminsäurealkylester der allgemeinen Formel (I) aus den einfach und billig herstellbaren Thiolcarbaminsäurealkenylestern durch Hydrieren gewonnen werden können. Über die Hydrierung von Thiolcarbaminsäurealkenylestern zu Alkylestern ist bisher in der Literatur noch nicht berichtet worden.

Gegenstand der Erfindung ist demnach ein Verfahren zur Herstellung von Thiolcarbaminsäureestern der allgemeinen Formel (I)

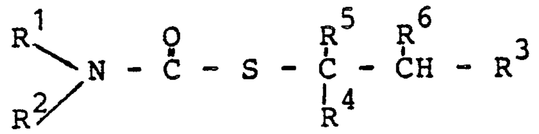
(I)

worin die Bedeutung von $R^1$, $R^2$, $R^3$, $R^4$, $R^5$ und $R^6$ die gleiche wie oben ist. Für das erfindungsgemäße Verfahren ist kennzeichnend, daß man

a) Thiocarbamatsalze der allgemeinen Formel (II)

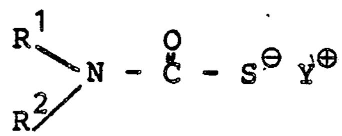
(II)

worin die Bedeutung von $R^1$ und $R^2$ die gleiche wie oben ist und

Y ein primäres, sekundäres oder tertiäres Ammoniumion oder ein Alkalimetallion bedeutet oder

b) Amine der allgemeinen Formel (III)

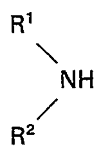
(III)

worin die Bedeutung von $R^1$ und $R^2$ die gleiche wie oben ist, zusammen mit Kohlenmonoxyd und Schwefel, oder

c) Amine der allgemeinen Formel (III) zusammen mit Carbonylsulfid

mit Alkinen der allgemeinen Formel (IV)

$$R^3—C\equiv C—R^4 \qquad (IV)$$

worin die Bedeutung von $R^3$ und $R^4$ die gleiche wie oben ist, umsetzt, und gewünschtenfalls die entstandene Verbindung der allgemeinen Formel (I), worin die Bedeutung von $R^1$, $R^2$, $R^3$ und $R^4$ die gleiche wie oben ist und $R^5$ zusammen mit $R^6$ eine chemische Bindung bildet, zu einer Verbindung der allgemeinen Formel (I), in der $R^5$ und $R^6$ für je ein Wasserstoffatom stehen, hydriert.

Gemäß der Variante a) wird das Thiolcarbaminsäuresalz der allgemeinen Formel (II) in Lösung mit dem entsprechenden 1-Alkin umgesetzt. Die Lösung wird vorzugsweise mit einem niederen Alkohol, zum Beispiel Methanol, Äthanol, n- oder i-Propanol, n-, sec.- oder tert.-Butanol, vorzugsweise jedoch Methanol, oder einem inerten Lösungsmittel, zum Beispiel Tetrahydrofuran, bereitet. Diese Lösung wird mit dem entsprechenden Alkin bei Drucken von 0,1—10 MPa und Temperaturen von 80—200°C, vorzugsweise 100—160°C, umgesetzt. Die Reaktion dauert 2—10 Stunden.

Gemäß einer bevorzugten Ausführung des Verfahrens a) wird aus dem Thiolcarbaminsäuresalz der allgemeinen Formel (II) eine möglichst konzentrierte Lösung bereitet. Für die Erzielung guter Ausbeuten hat sich eine Konzentration von wenigstens 30 Gew.-% in der Lösung als vorteilhaft erwiesen.

Die Verfahren b) und c) sind hinsichtlich ihrer Bedingungen ähnlich. Die Reaktionstemperatur kann innerhalb eines weiten Bereiches, zwischen 25 und 200°C, vorzugsweise 80 und 160°C, gewählt werden. Es ist nicht erforderlich, die Temperatur auf einem konstanten Wert zu halten. So kann man gemäß a) zum Beispiel das Amin, das Kohlenmonoxyd und den Schwefel bei etwa 100°C umsetzen und dann das Reaktionsgemisch — abhängend von der Reaktionsfähigkeit des Alkins — kühlen oder erwärmen und dann mit dem Alkin umsetzen. Die Reaktionszeit hängt von Menge und Reaktivität der Reaktionskomponenten und von der Temperatur ab und liegt zwischen 30 Minuten und 30 Stunden.

Die Reihenfolge der Zugabe der Reaktanten ist im allgemeinen nicht kritisch. Bevorzugt geht man so vor, daß man die erforderliche Menge Amin und Schwefel in den Reaktor gibt, den Reaktor verschließt und

dann den Gasraum mit Acetylen spült und schließlich den Acetylen- und Kohlenmonoxyddruck einstellt. Die Zugabe der Komponenten kann kontinuierlich oder diskontinuierlich erfolgen.

Das Molverhältnis zwischen Amin und Schwefel beträgt im allgemeinen 5:1 bis 1:5. Um eine möglichst vollständige Umsetzung des Amins zu erreichen, ist es bevorzugt, den Schwefel im Überschuß einzusetzen. Das Molverhältnis zwischen Kohlenmonoxyd und Schwefel beträgt zweckmäßig zwischen 1:1 und 3:1. Das Molverhältnis zwischen Alkin und Amin schließlich kann in einem grösseren Bereich frei gewählt werden und liegt etwa zwischen 1:2 und 100:1. Niedere Alkine, zum Beispiel Acetylen, verursachen in der erforderlichen Menge im Reaktor einen Druck von 0,1—5 MPa.

Die Reaktion kann in Gegenwart eines Lösungsmittels oder ohne ein solches vorgenommen werden. Als Lösungsmittel kommen zum Beispiel folgende in Frage: niedere Alkanole, zum Beispiel Methanol, Äthanol, Propanol, Isopropanol, n-, Iso-, tert.- und sec.-Butanol, vorzugsweise Methanol; ferner dipolaraprotische Lösungsmittel wie Acetonitril, Benzonitril, Dimethylformamid, Dimethylsulfoxyd, Hexamethylphosphorsäuretriamid; sonstige inerte Lösungsmittel, wie Äther, zum Beispiel Tetrahydrofuran, Dioxan, Aceton, tertiäre aliphatische Amine, Pyridin und seine Homologen, aromatische Kohlenwassserstoffe, chlorierte Kohlenwasserstoffe, Carbonsäuren, carbonsäureester.

In manchen Fällen kann die Reaktion auch in der Schmelze, d.h. ohne Lösungsmittel ausgeführt werden.

Bei der Synthese der unterschiedlichen Thiolcarbaminsäure-S-alkenylester kann häufig beobachtet werden, daß gegen Ende der Umsetzung die Geschwindigkeit der Vinylierungsreaktion absinkt. In diesen Fällen ist es vorteilhaft, zur Verkürzung der Reaktionszeit und einer möglichst völligen Ausnutzung des Amins das noch nicht umgesetzte Salz in geeigneter Weise zu alkylieren. Diese Verfahrensweise ist besonders empfehlenswert, wenn der hergestellte Alkenylester ohnehin zum Alkylester umgesetzt werden soll. Zur Herstellung von S-Vinylestern wird demnach das nicht völlig umgesetzte Reaktionsgemisch zum Beispiel mit Diäthylsulfat oder Äthylchlorid behandelt. Das erhaltene Gemisch aus Alkenyl- und Alkylester wird dann der weiteren Verarbeitung unterzogen.

In einem letzten, fakultativen Schritt des Verfahrens können die erhaltenen S-Alkenylester der allgemeinen Formel (I) $R^5$ und $R^6$ zusammen chemische Bindung) gewünschtenfalls zu den entsprechenden S-Alkylestern ($R^5 = R^6 = H$) hydriert werden. Die Hydrierung kann als katalytische Transfer-Hydrierung, d.h. in Gegenwart eines Katalysators und einer wasserstoffabgebenden Verbindung (Wasserstoffdonor) vorgenommen werden, jedoch ist auch die gewöhnliche katalytische Hydrierung mit Gas anwendbar.

Wird gemäß der katalytischen Transfer-Hydrierung (C. Brieget, T. I. Westrick: Catalytic Transfer Hydrogenation, Chem. Rev. *74* (5), 567/1974/) gearbeitet, so wird die ungesättigte Verbindung in einem geeigneten Lösungsmittel in Gegenwart eines Wasserstoffdonors und in Gegenwart eines Katalysators hydriert. Statt des Lösungsmittels kann auch der Überschuß des Wasserstoffdonors verwendet werden. Die Transfer-Hydrierung kann unter inerter, wasserstofffreier Gasatmosphäre vorgenommen werden, jedoch ist es vorteilhafter, unter Wasserstoffdruck, bevorzugt 0,1—5 MPa, zu arbeiten.

Für die katalytische Transfer-Hydrierung kommen folgende Katalysatoren in Frage: gegebenenfalls auf einen Träger aufgebrachtes Palladium oder Platin, zum Beispiel 10 %ige Palladiumaktivkohle oder ein 10 %iger Palladiumkatalysator auf einem Kalksteinträger oder ein 0,1 %iger Palladiumkatalysator auf Aluminiumoxyd, ferner Platinschwarz, Platin- und Palladium-halogenide oder -oxyde ($PdCl_2$, $PtO_2$), ferner Raneynickel; Ruthenium, Iridium, Rhodium enthaltende Komplexe, zum Beispiel $RuCl_2(Ph_3P)_3$, $IrHCl_2(Mo_2SO_4)$, $IrBr(CO)(Ph_3P)_3$ oder $RhCl(Ph_3P)_3$.

Besonders bevorzugt wird als Lösungsmittel und gleichzeitig Wasserstoffdonor ein niederer Alkohol, zum Beispiel Methanol, Äthanol, n- oder i-Propanol, n-, i-, sec.- oder tert.-Butanol verwendet.

Die ungesättigten Verbindungen der allgemeinen Formel (I) können auch direkt katalytisch hydriert werden. In diesem Fall ist das Verhältnis zwischen Substrat und Katalysator besonders günstig. Die direkte Hydrierung wird in einem druckfesten Gefäß, bei Temperaturen zwischen 150 und 300°C und Drucken von 0,1—10 MPa, vorzugsweise 1—5 MPa, ausgeführt. Als Lösungsmittel kommen die für die katalytische Transfer-Hydrierung genannten, ferner auch sonstige protische Lösungsmittel, zum Beispiel Eisessig, in Frage. Katalysiert wird mit den üblichen Hydrierkatalysatoren, zum Beispiel Raney-Nickel, Palladium und Platin, gegebenenfalls auf einen Träger aus Aluminiumoxyd, Silikagel, Aktivkohle oder Zeolith aufgebracht. Wesentlich ist lediglich, daß der Katalysator gegen Schwefel nicht empfindlich ist.

Das erfindungsgemäße Verfahren wird im folgenden durch Ausführungsbeispiele näher erläutert, ist jedoch nicht auf die Beispiele beschränkt.

## Beispiel 1

Verfahren a)

In einem aus säurefestem Stahl gefertigten Autoklav mit 250 $cm^3$ Nutzvolumen werden in 100 $cm^3$ Methanol 50 g (0,19 Mol) N,N-Di-(n-propyl)-thiolcarbaminsäure-di-(n-propyl)-ammoniumsalz gelöst. Der Reaktor wird verschlossen, der Gasraum mit Acetylen durchspült und dann mit Acetylen des Druckes 1,5 MPa gefüllt. Der Autoklav wird unter intensivem Rühren einige Stunden lang bei 130°C gehalten. Dann läßt man abkühlen, bläst den Druck ab, gießt das Reaktionsgemisch in ein fünffaches Volumen Wasser und extrahiert dieses mit Chloroform. Die organische Phase wird über Magnesiumsulfat getrocknet und unter vermindertem Druck eingedampft. Der Rückstand wird destilliert. Man erhält 23 g (61,2%) N,N-Di-(n-propyl)-thiolcarbaminsäure-vinylester. Siedepunkt: 105—112°C/6 mbar.

Beispiel 2

Verfahren a)

Man arbeitet auf die im Beispiel 1 beschriebene Weise, verwendet als Ausgangsverbindung jedoch N,N-Diäthyl-thiolcarbaminsäure-triäthylammoniumsalz. Nach Destillation bei vermindertem Druck erhält man 19 g (41%) N,N-Diäthyl-thiolcarbaminsäure-vinylester.

Beispiel 3

Verfahren b)

In einen mit Rührer und Innenheizung ausgerüsteten Reaktor aus rostfreiem Stahl (Nutzvolumen 1000 $cm^3$) werden 9,6 g (0,3 g-Atom) Schwefel, 82 $cm^3$ (0,6 Mol) Di-n-propylamin und 100 $cm^3$ Methanol eingefüllt. Der Reaktor wird mit Aceton ausgespült. Dann wird der partialdruck des Acetylens und anschließend auch der partialdruck des Kohlenmonoxyds auf 1,72 MPa eingestellt. Der Rührer wird eingeschaltet und der Reaktor auf 130°C aufgeheizt. Diese Temperatur wird über 9,5 Stunden lang eingehalten. Dann wird der Reaktor abgekühlt und druckfrei gemacht. Das Reaktionsgemisch wird mit 100 $cm^3$ Wasser und dann die wäßrige Phase mit 3 × 50 $cm^3$ Chloroform extrahiert. Die organischen Phasen werden vereinigt, über Magnesiumsulfat getrocknet, filtriert, eingedampft und der Rückstand im Vakuum destilliert. Man erhält 26,4 g (47%) N,N-Di-(n-propyl)-thiolcarbaminsäure-S-vinylester. Siedepunkt: 115—120°C/12—13 mbar.

Beispiel 4

Verfahren b)

In den Reaktor gemäß Beispiel 2 werden 9,6 g (0,3 g-Atom) Schwefel, 82 $cm^3$ (0,6 Mol) Di-n-propylamin und 100 $cm^3$ Methanol gefüllt. Der Reaktor wird mit Kohlenmonoxyd durchspült, dann wird ein CO-Druck von 1,9 MPa eingestellt. Nach Einschalten des Rührwerkes wird der Reaktor auf 130°C aufgeheizt und bei dieser Temperatur 90 Minuten lang gehalten. Nach dem Abkühlen und Abblasen wird der Reaktor mit Acetylen bis zu einem Druck von 1,9 MPa aufgefüllt. Nach 5,5 Stunden Reaktionszeit bei 130°C erhält man (Aufarbeitung wie im Beispiel 2) 23,9 g (46%) N,N-Di-(n-propyl)-thiolcarbaminsäure-S-vinylester, dessen Siedepunkt mit dem der gemäß Beispiel 2 erhaltenen Verbindung übereinstimmt.

Beispiel 5

Verfahren b)

In den Reaktor gemäß Beispiel 2 werden 37,3 g (1,17 g-Atom) Schwefel, 100 $cm^3$ (0,67 Mol) N-Äthylcyclohexylamin und 100 $cm^3$ Methanol gefüllt. Der Gasraum des Reaktors wird gespült, dann wird ein Acetylen-Druck von 1,4 MPa und ein CO-Druck von 3,72 eingestellt. Der Reaktor wird aufgeheizt und bei 100° für eine Stunde thermostatisiert. Dann wird der Reaktor auf 120°C aufgeheizt und diese Temperatur 8,5 Stunden lang eingehalten. Wie die gaschromatographische Analyse ausweist, ist bei 40 %igem Umsatz der N-Äthyl-N-cyclohexyl-thiolcarbaminsäure-S-vinylester in 25 %iger Ausbeute entstanden. Siedepunkt: 122—124°C/2,6 mbar.

Beispiel 6

Katalytische Transfer-Hydrierung

In einen aus säurefestem Stahl gefertigten, druckfesten, luftdicht verschließbaren Reaktor von 250 $cm^3$ Volumen werden 100 $cm^3$ Methanol eingefüllt und 2 g (12,5 mMol) N,N-Diäthylthiolcarbaminsäure-vinylester darin gelöst. Der Reaktor wird mit Wasserstoff gespült und dann mit Wasserstoff von 1 MPa Druck gefüllt. Das Gemisch wird auf 50°C erwärmt und unter Rühren innerhalb von 4 Stunden mit 5 × 0,1 g 10 %iger Palladiumaktivkohle versetzt. Das Gemisch wird noch zwei Stunden lang gerührt und dann filtriert. Das Filtrat wird eingedampft und der Rückstand unter vermindertem Druck destilliert. Man erhält 1,4 g (69,1%) N,N-Diäthylthiolcarbaminsäure-äthylester; Siedepunkt: 35—38°C/8 Hgmm.

Auf die gleiche Weise, jedoch mit anderen Katalysatoren werden folgende Ergebnisse erzielt:

| Katalysator | Ausbeute, % |
|---|---|
| Raney-Nickel | 72 |
| 10 %ige Platinaktivkohle | 58,3 |
| Platinschwarz | 78,6 |
| $RuCl_2(Ph_2P)_3$ | 22,1 |
| $IrBr(CO)(Ph_3P)_3$ | 35,2 |

Beispiel 7

Katalytische Transfer-Hydrierung

In dem Reaktor gemäß Beispiel 5 werden in 100 $cm^3$ Methanol 2 g (10,6 mMol) N,N-Di-(n-propyl)-thiolcarbaminsäure-vinylester gelöst. Der Gasraum wird mit Wasserstoff gespült und dann mit Wasserstoff unter 10 MPa Druck gefüllt. Zu dem auf 50°C erwärmten Reaktionsgemisch gibt man innerhalb von 4 Stunden 5 × 0,1 g Raney-Nickel. Nach Beendigung der Zugabe wird noch zwei Stunden lang gerührt, dann wird der Katalysator abfiltriert, das Filtrat eingedampft und der ölige Rückstand unter vermindertem Druck destilliert. Man erhält 1,72 g (85,1%) N,N-Di-(n-propyl)-thiolcarbaminsäureäthylester; Siedepunkt: 80—82°C/8 Hgmm.

Beispiel 8

Direkte Hydrierung

In einem druckfesten Behälter von 300 $cm^3$ Volumen werden in 100 $cm^3$ Methanol 20 $cm^3$ N,N-Di-(n-propyl)-thiolcarbaminsäure-vinylester gelöst. Zu der Lösung gibt man 1 g 1 %ige Palladiumaktivkohle. Das Gefäß wird verschlossen, dreimal mit Wasserstoff gespült und dann mit Wasserstoff unter 1,5 MPa Druck gefüllt. Das Gemisch wird bei 240°C 8 Stunden lang hydriert. Man erhält den N,N-Di-(n-propyl)-thiolcarbaminsäureäthylester in 82 %iger Ausbeute. 12% der Ausgangsverbindung werden zurückgewonnen.

Auf ähnliche Weise werden die in der folgenden Tabelle aufgeführten Verbindungen hergestellt.

| Verbindungen der allgemeinen Formel (I) mit $R^3 = R^4 = R^5 = R^6 = H$ | | | | | |
|---|---|---|---|---|---|
| $R^1$ | $R^2$ | Katalysator | t(h) | T°C | Ausbeute, % |
| n-Propyl | n-Propyl | Raney-Ni | 4 | 200 | 100 |
| Äthyl | Cyclohexyl | Pd auf $\gamma \cdot Al_2O_3$ | 4 | 240 | 100 |
| Äthyl | n-Butyl | Pd auf $\gamma \cdot Al_2O_3$ | 4 | 240 | 100 |
| $-(CH_2)_6-$ | | Pd auf $\gamma \cdot Al_2O_3$ | 5 | 240 | 100 |

Beispiel 9

Verfahren b)

In einen Reaktor mit dem Volumen 1000 $cm^3$ werden 148 $cm^3$ (1,2 Mol) Diallylamin und 57,6 g (1,8 g-Atom) Schwefel vorgelegt. Der Reaktor wird mit Kohlenmonoxyd des Druckes 5,9 MPa gefüllt. Nach einer Stunde Reaktion bei 100°C wird in den Reaktor Acetylen komprimiert. Unter mehrmaligem Auffüllen mit Acetylen wird die Vinylierung bei 120°C noch 8 Stunden lang fortgesetzt. Man erhält den N,N-Diallyl-thiolcarbaminsäure-S-vinylester in 10 %iger Ausbeute.

Beispiel 10

Verfahren c)

In einen Reaktor mit 300 $cm^3$ Nutzvolumen werden 25 $cm^3$ (0,18 Mol) Dipropylamin, 31,25 g (0,52 Mol) Carbonylsulfid und 50 $cm^3$ Methanol eingefüllt, dann wird der Acetylendruck auf 1,7 MPa eingestellt. Der Reaktor wird auf 130—140°C aufgeheizt und 10 Stunden lang bei dieser Temperatur belassen. Nach dem Abkühlen des Reaktors und dem Abblasen der Gasphase wird das rohe Gemisch einer Vakuumdestillation unterzogen. Der Dipropylthiolcarbaminsäure-S-vinylester wird bezogen auf das Dipropylamin in einer Menge von 24,3 g (71%) erhalten.

Beispiel 11

Nachträgliche Alkylierung

In einen Reaktor von 1000 $cm^3$ Volumen werden 56 g (1,75 g-Atom) Schwefel und 164 $cm^3$ (1,2 Mol) Di-(n-propyl)-amin eingefüllt, und nach dem Spülen des Reaktors mit Acetylen wird der Gasdruck des Acetylens auf 1,7 MPa, der des Kohlenmonoxyds auf 5,9 MPa eingestellt. Das Gemisch wird bei 100°C 75 Minuten lang, dann bei 120°C 14 Stunden lang umgesetzt. Zeitweise wird Acetylen nachgefüllt.

Das Reaktionsgemisch wird in einen Dreihalskolben von 1 $dm^3$ Volumen gefüllt, mit 400 $cm^3$ Dioxan verdünnt und dann unter Rühren tropfenweise mit 17,8 $cm^3$ (0,22 Mol) Äthyljodid versetzt. Der Niederschlag wird durch Filtrieren entfernt. Die flüchtigen Komponenten des Filtrats werden verdampft, der Rückstand einer Vakuumdestillation unterzogen. Die Hauptfraktion geht bei 80—98°C und einem Druck von 1 mbar über. Diese Fraktion (148 g) besteht zu 20% aus Dipropyl-thiolcarbaminsäure-S-äthylester und zu 80% aus Dipropyl-thiolcarbaminsäure-S-vinylester. Das entspricht einer auf Dipropylamin bezogen 66 %igen Ausbeute, die Konversion ist 71%.

**Patentansprüche**

1. Verfahren zur Herstellung von Thiolcarbaminsäureestern der allgemeinen Formel (I)

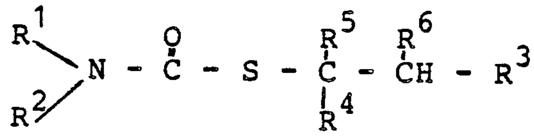 (I)

worin

$R^1$ und $R^2$ unabhängig voneinander für Wasserstoff oder eine gerade oder verzweigte Alkylgruppe mit 1—6 Kohlenstoffatomen oder Alkenylgruppe mit 2—6 Kohlenstoffatomen oder für durch Halogen, Sauerstoff, Schwefel und/oder Stickstoff ein- oder mehrfach substituierte Alkylgruppe mit 1—6 beziehungsweise Alkenylgruppe mit 2—6 Kohlenstoffatomen stehen oder zusammen eine gegebenenfalls substituierte α,ω-Alkylengruppe mit 4—6 Kohlenstoffatomen bilden,

$R^3$ und $R^4$ unabhängig voneinander für Wasserstoff oder eine Alkylgruppe mit 1—4 Kohlenstoffatomen stehen und letztere durch Halogen oder eine Sauerstoff, Schwefel und/oder Stickstoff enthaltende Gruppe substituiert sein kann, und

$R^5$ und $R^6$ beide für Wasserstoff stehen oder gemeinsam eine chemische Bindung bilden, dadurch gekennzeichnet, daß man

A) thiolcarbamatsalze der allgemeinen Formel (II)

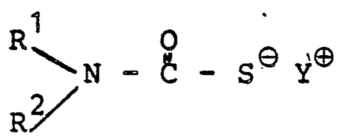 (II)

worin die Bedeutung von $R^1$ und $R^2$ die gleiche wie oben ist und

Y ein primäres, sekundäres oder tertiäres Ammoniumion oder ein Alkalimetallion bedeutet, oder

b) Amine der allgemeinen Formel (III)

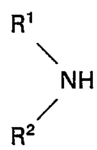 (III)

worin die Bedeutung von $R^1$ und $R^2$ die gleiche wie oben ist, zusammen mit Kohlenmonoxyd und Schwefel, oder

c) Amine der allgemeinen Formel (III) zusammen mit Carbonylsulfid

mit Alkinen der allgemeinen Formel (IV)

$$R^3—C≡C—R^4 \quad (IV)$$

worin die Bedeutung von $R^3$ und $R^4$ die gleiche wie oben ist, umsetzt und gewünschtenfalls (gegebenenfalls nach Zusatz eines Alkylierungsmittels) die entstandene Verbindung der allgemeinen Formel (I), worin die Bedeutung von $R^1$, $R^2$, $R^3$ und $R^4$ die gleiche wie oben ist und $R^5$ zusammen mit $R^6$ eine chemische Bindung bildet, zu einer Verbindung der allgemeinen Formel (I), in der $R^5$ und $R^6$ für je ein Wasserstoffatom stehen, hydriert.

2. Verfahren nach Anspruch 1a), dadurch gekennzeichnet, daß man die Umsetzung in einer mehr als 30 %igen Lösung des Thiolcarbamatsalzes ausführt.

3. Verfahren nach Anspruch 1a), dadurch gekennzeichnet, daß man die Umsetzung in der Schmelze des Thiolcarbamatsalzes ausführt.

4. Verfahren nach Anspruch 1a), dadurch gekennzeichnet, daß man die Umsetzung bei Temperaturen von 80—200°C, vorzugsweise 100—160°C, vornimmt.

5. Verfahren nach Anspruch 1b) oder 1c), dadurch gekennzeichnet, daß man die Reaktion bei Temperaturen von 25—200°C, vorzugsweise 80—160°C, vornimmt.

6. Verfahren nach Anspruch 1b) oder 1c), dadurch gekennzeichnet, daß man den Schwefel beziehungsweise des Carbonylsulfid bezogen auf das Amin im Überschuß einsetzt.

7. Verfahren nach einem der Ansprüche 1—6, dadurch gekennzeichnet, daß man die anschließende Hydrierung als katalytische Transfer-Hydrierung ausführt.

8. Verfahren nach einem der Ansprüche 1—6, dadurch gekennzeichnet, daß man die anschließende Hydrierung als direkte katalytische Hydrierung ausführt.

9. Verfahren nach Anspruch 7, dadurch gekennzeichnet, daß man die Umsetzung im Überschuß des Wasserstoffdonors der Transfer-Hydrierung als Lösungsmittel vornimmt.

10. Verfahren nach Anspruch 8, dadurch gekennzeichnet, daß man die Hydrierung unter einem Wasserstoffdruck von 0,1—10 MPa vornimmt.

11. Verfahren nach einem der Ansprüche 1—10, dadurch gekennzeichnet, daß man von N,N-Di-(n-propyl)-thiolcarbaminsäuresalzen und Acetylen oder von Di-(n-propyl)-amin, Schwefel, Kohlenmonoxyd und Acetylen ausgeht.

12. Verfahren nach einem der Ansprüche 1—10, dadurch gekennzeichnet, daß man von N,N-Diäthylthiolcarbaminsäuresalzen und Acetylen oder von Diäthylamin, Schwefel, Kohlenmonoxyd und Acetylen ausgeht.

13. Verfahren nach einem der Ansprüche 1—10, dadurch gekennzeichnet, daß man von N-Äthyl-N-cyclohexyl-amin, Schwefel, Kohlenmonoxyd und Acetylen ausgeht.

14. Verfahren nach einem der Ansprüche 1—10, dadurch gekennzeichnet, daß man von Di-(sec.-butyl)-amin, Schwefel, Kohlenmonoxyd und Acetylen ausgeht.

15. Verfahren nach einem der Ansprüche 1—10, dadurch gekennzeichnet, daß man von N-Äthyl-N-(n-butyl)-amin, Schwefel, Kohlenmonoxyd und Acetylen ausgeht.

16. Verfahren nach einem der Ansprüche 1—10, dadurch gekennzeichnet, dàß man von Perhydroazepin, Schwefel, Kohlenmonoxyd und Acetylen ausgeht.

17. Verfahren nach einem der Ansprüche 1—10, dadurch gekennzeichnet, daß man von 3-(Dimethylamino)-propylamin, Schwefel, Kohlenmonoxyd und Acetylen ausgeht.

18. Verfahren nach einem der Ansprüche 1—10, dadurch gekennzeichnet, daß man von Di-(n-propyl)-amin, Schwefel, Kohlenmonoxyd und 1-Propin ausgeht.

**Revendications**

1. Procédé de préparation de thiolcarbamates connus de formule générale I:

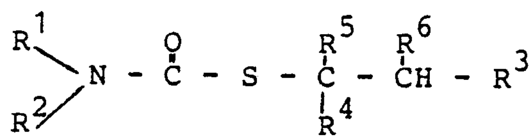

(I)

dans laquelle:

$R^1$ et $R^2$ représentent indépendamment l'un de l'autre un atome d'hydrogène ou un radical alkyle à chaîne droite ou ramifiée renfermant de 1 à 6 atomes de carbone, ou un groupe alkényle renfermant de 2 à 6 atomes de carbone, ou un groupe alkyle renfermant de 1 à 6 atomes de carbone et/ou un groupe alkényle renfermant de 2 à 6 atomes de carbone, substitués une ou plusieurs fois par un atome d'halogène, d'oxygène, de soufre et/ou d'azote ou ils forment ensemble un groupe α,ω-alkylène, éventuellement substitué, renfermant de 4 à 6 atomes de carbone;

$R^3$ et $R^4$ représentent indépendamment l'un de l'autre un atome d'hydrogène ou un groupe alkyle renfermant de 1 à 4 atomes de carbone et ce dernier peut être substitué par un atome d'halogène ou un groupe contenant un atome d'oxygène, de soufre et/ou d'azote; et

$R^5$ et $R^6$ représentent tous les deux un atome d'hydrogène ou forment ensemble une liaison chimique,

caractérisé en ce que:

a) des sels de thiolcarbamates de formule générale (II):

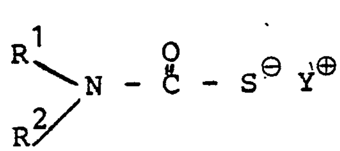

(II)

dans laquelle: la signification de $R^1$ et $R^2$ est la même que ci-dessus; et

Y représente un ion ammonium primaire, secondaire ou tertiaire ou un ion de métal alcalin, ou

b) des amines de formule générale (III):

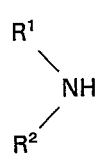

(III)

dans laquelle la signification de $R^1$ et $R^2$ est la même que ci-dessus, en même temps que du monoxyde de carbone et du soufre, ou

c) des amines de formule générale (III) en même temps que du sulfure de carbonyle, on fait réagir avec des alkynes de formule générale (IV):

$$R^3—C≡C—R^4 \qquad (IV)$$

dans laquelle: la signification de $R^3$ et $R^4$ est la même que ci-dessus, et, le cas échéant (éventuellement après addition d'un agent d'alkylation), on hydrogène le dérivé formé de formule générale, (I) dans laquelle la signification de $R^1$, $R^2$, $R^3$ et $R^4$ est la même que ci-dessus et $R^5$ en même temps que $R^6$ forment une liaison chimique, en un dérivé de formule générale (I), dans laquelle $R^5$ et $R^6$ représentent chacun un atome d'hydrogène.

2. Procédé selon la revendication 1a), caractérisé en ce que l'on met la réaction en oeuvre dans une solution comprenant plus de 30% du sel de thiolcarbamate.

3. Procédé selon la revendication 1a), caractérisé en ce que l'on met la réaction en oeuvre dans une masse fondue du sel de thiocarbamate.

4. Procédé selon la revendication 1a), caractérisé en ce que l'on met la réaction en oeuvre à des températures de 80 à 200°C, de préférence de 100 à 160°C.

5. Procédé selon la revendication 1b) ou 1c), caractérisé en ce que l'on met la réaction en oeuvre à des températures de 25 à 200°C, de préférence de 80 à 160°C.

6. Procédé selon la revendication 1b) ou 1c), caractérisé en ce que l'on utilise un excès de soufre et/ou de sulfure de carbonyle par rapport à l'amine.

7. Procédé selon l'une quelconque des revendications 1 à 6, caractérisé en ce que l'hydrogénation ultérieure est une hydrogénation par transfert catalytique.

8. Procédé selon l'une quelconque des revendications 1 à 6, caractérisé en ce que l'hydrogénation ultérieure est une hydrogénation catalytique directe.

9. Procédé selon la revendication 7, caractérisé en ce que l'on met la réaction en oeuvre en présence d'un excès de donneur d'hydrogène de l'hydrogénation par transfert servant de solvant.

10. Procédé selon la revendication 8, caractérisé en ce que l'on met l'hydrogénation en oeuvre sous une pression d'hydrogène de 0,1 à 10 MPa.

11. Procédé selon l'une quelconque des revendications 1 à 10, caractérisé en ce que l'on part de sels d'acide N,N-di-(n-propyl)thiolcarbamique et d'acétylène ou de di-(n-propyl)-amine, de soufre, de monoxyde de carbone et d'acétylène.

12. Procédé selon l'une quelconque des revendications 1 à 10, caractérisé en ce que l'on part de sels d'acide N,N-diéthylthiolcarbamique et d'acétylène ou de diéthylamine, de soufre, de monoxyde de carbone et d'acétylène.

13. Procédé selon l'une quelconque des revendications 1 à 10, caractérisé en ce que l'on part de N-éthyl-N-cyclohexylamine, de soufre, de monoxyde de carbone et d'acétylène.

14. Procédé selon l'une quelconque des revendications 1 à 10, caractérisé en ce que l'on part de di-(butyl-secondaire)-amine, de soufre, de monoxyde de carbone et d'acétylène.

15. Procédé selon l'une quelconque des revendications 1 à 10, caractérisé en ce que l'on part de N-éthyl-N-(n-butyl)-amine, de soufre, de monoxyde de carbone et d'acétylène.

16. Procédé selon l'une quelconque des revendications 1 à 10, caractérisé en ce que l'on part de perhydroazépine, de soufre, de monoxyde de carbone et d'acétylène.

17. Procédé selon l'une quelconque des revendications 1 à 10, caractérisé en ce que l'on part de 3-(diméthylamino)-propylamine, de soufre, de monoxyde de carbone et d'acétylène.

18. Procédé selon l'une quelconque des revendications 1 à 10, caractérisé en ce que l'on part de di-(n-propyl)amine, de soufre, de monoxyde de carbone et de propyne-1.

**Claims**

1. A process for preparing thiolcarbamine acid esters having the general formula (I)

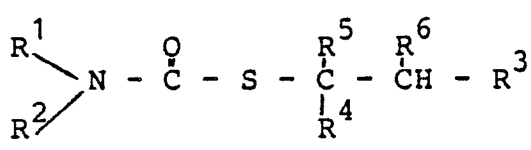

(I)

wherein

$R^1$ and $R^2$ are independently hydrogen or a straight-chain or a branched-chain alkyl group having 1—6 carbon atoms or alkenyl group having 2—6 carbon atoms or an alkyl group being singly or multiply substituted with halogen, oxygen, sulphur and/or nitrogen and having 1—6 carbon atoms, or an alkenyl group being singly or multiply substituted with halogen, oxygen, sulphur and/or nitrogen and having 2—6 carbon atoms, respectively, or together form an substituted or unsubstituted α,ω-alkylen group having 4—6 carbon atoms;

$R^3$ and $R^4$ are independently hydrogen or an alkyl group having 1—4 carbon atoms, the latter of which may be substituted by halogen or a group containing oxygen, sulphur and/or nitrogen; and

$R^5$ and $R^6$ are both hydrogen or form together a chemical bond,

characterized by contacting

a) thiol carbamate salts having the general formula (II)

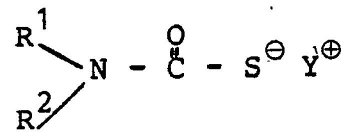 (II)

wherein $R^1$ and $R^2$ are as defined above and

Y is a primary, secondary or tertiary ammonium ion or an alkali metal ion; or

b) amines of the general formula (III)

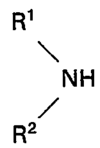 (III)

wherein $R^1$ and $R^2$ are as defined above, together with carbon monoxide and sulphur; or

c) amines of the general formula (III) together with carbonyl sulfide;

with alkines of the general formula (IV)

$$R^3—C{\equiv}C—R^4 \qquad (IV)$$

wherein $R^3$ and $R^4$ are as defined above and, if desired (optionally after the addition of an alkylating agent) hydrogenating the resulting compound of the general formula (I), wherein $R^1$, $R^2$, $R^3$ and $R^4$ are as defined above and $R^5$ together with $R^6$ forms a chemical bond, to a compound of the general formula (I), wherein $R^5$ and $R^6$ are each one hydrogen atom.

2. The process according to claim 1a), characterized in that the reaction is carried out in solution containing more than 30% of said thiolcarbamate salt.

3. The process according to claim 1a), characterized in that the reaction is carried out in a molten thiolcarbamate salt.

4. The process according to claim 1a), characterized in that the reaction is carried out at temperatures ranging from 80 to 200°C, preferably from 100 to 160°C.

5. The process according to claim 1b) or 1c), characterized in that the reaction is carried out at temperatures ranging from 25 to 200°C, preferably from 80 to 160°C.

6. The process according to claim 1b) or 1c), characterized in that the sulphur or the carbonyl sulfonide, respectively, is used in an excess, related to the amine.

7. The process according to one of the claims 1 to 6, characterized in that the subsequent hydrogenation is carried out in the form of a catalytic transfer hydrogenation.

8. The process according to one of the claims 1 to 6, characterized in that the subsequent hydrogenation is carried out in the form a direct catalytic hydrogenation.

9. The process according to claim 7, characterized in that the reaction is carried out in an excess of the hydrogen donor used as solvent in the transfer hydrogenation.

10. The process according to claim 8, characterized in that the hydrogenation is carried out under a hydrogen pressure of from 0.1 to 10 MPa.

11. The process according to one of the claims 1 to 10, characterized by using as the starting materials N,N-Di-(n-propyl)-thiolcarbamine acid salts and acetylene or Di-(n-propyl)-amine, sulphur, carbon monoxide and acetylene.

12. The process according to one of the claims 1 to 10, characterized by using as the starting materials N,N-diethyl-thiolcarbamine acid salts and acetylene or diethylamine, sulphur, carbon monoxide and acetylene.

13. The process according to one of the claims 1 to 10, characterized by using as the starting materials N-ethyl-N-cyclohexylamine, sulphur, carbon monoxide and acetylene.

14. The process according to one of the claims 1 to 10, characterized by using Di-(sec.-butyl)-amine, sulphur, carbon monoxide and acetylene as the starting materials.

15. The process according to one of the claims 1 to 10, characterized by using as the starting materials N-ethyl-N-(n-butyl)-amine, sulphur, carbon monoxide and acetylene.

16. The process according to one of the claims 1 to 10, characterized by using as the starting materials perhydroazepine, sulphur, carbon monoxide and acetylene.

17. The process according to one of the claims 1 to 10, characterized by using as the starting materials 3-(dimethylamino)-propylamine, sulphur, carbon monoxide and acetylene.

18. The process according to one of the claims 1 to 10, characterized by using as the starting materials Di-(n-propyl)-amine, sulphur, carbon monoxide and 1-propine.